# EUROPÄISCHE PATENTSCHRIFT

(11) **EP 1 051 120 B1**
(45) Veröffentlichungstag und Bekanntmachung des Hinweises auf die Patenterteilung: **06.05.2004**
(21) Anmeldenummer: 99907374.5
(22) Anmeldetag: 15.01.1999
(51) Int. Cl.: A61B 18/12

(54) **BIPOLARES KOAGULATIONS- UND SCHNEIDGERÄT FÜR DIE ENDOSKOPISCHE CHIRURGIE**
BIPOLAR COAGULATION AND CUTTING DEVICE FOR ENDOSCOPIC SURGERY
APPAREIL D'INCISION ET DE COAGULATION BIPOLAIRE POUR CHIRURGIE ENDOSCOPIQUE

(30) Priorität: 26.01.1998 DE 19802743
(43) Veröffentlichungstag der Anmeldung: 15.11.2000
(73) Patentinhaber: Forschungszentrum Karlsruhe GmbH, 76133 Karlsruhe (DE)
(72) Erfinder: TRAPP, Rainer, D-76676 Graben-Neudorf (DE); DUX, Uwe, D-76474 Au am Rhein (DE)
(86) Internationale Anmeldenummer: PCT/EP1999/000214
(87) Internationale Veröffentlichungsnummer: WO 1999/037228

(56) Entgegenhaltungen:
- WO-A-95/13027
- DE-A- 4 032 471
- US-A- 5 718 703

## Beschreibung

Die Erfindung betrifft ein bipolares Koagulations- und Schneidegerät für die endoskopische Chirurgie.

Geräte dieser Art sind Werkzeuge für den Chirurgen, Gewebe zu greifen, bipolar zu koagulieren und anschließend durch den umfaßten Bereich zu durchtrennen.

Ein solches kombiniertes Instrument ist aus auf dem 1995 verbreiteten Informationsblatt der Cabot Technology Corporaten, 2150 Cabot Boulevard West, Langhorne, PA 19047 USA bekannt. Das Instrument hat eine Greifeinrichtung, die aus zwei Branchen besteht, mit der entlang der u-förmigen Greifkontur Gewebe koaguliert wird. Anschließend wird ein Messer durch die Greif- und Koagulationseinrichtung vorgeschoben, das zwischen dem koagulierten Bereich durchtrennt. Ein vom Prinzip her gleichartig zu betätigendes Kombinationsinstrument wird in der US 5,445,638 beschrieben. Beiden Instrumenten ist gemeinsam, daß mit der Greifeinrichtung auch bipolar koaguliert wird und anschließend der Zwischenbereich mit einem mechanischen Mittel, nämlich einem Messer, durchtrennt wird. Das ist durch den handhabenden Chirurgen mit einem über das gesamte Instrument zu übertragenden Kraftaufwand durchzuführen.

Der Erfindung liegt die Aufgabe zugrunde, ein kombiniertes Instrument für die endoskopische Chirurgie bereitzustellen, mit dem sicher Gewebe gegriffen und gehalten werden kann, mit dem entsprechend der Branchenform der Zange gleichzeitig eine zuverlässige Linienkoagulation durchgeführt werden kann und schließlich in Vorwärtsrichtung im Anschluß an die Koagulation vollends im koagulierten Zwischenbereich eine Gewebedurchtrennung im Zangenbereich sicher und mit allenfalls minimalem Kraftaufwand möglich ist.

Die Aufgabe wird durch ein bipolares Koagulations- und Schneidegerät mit den Merkmalen des Anspruch 1 gelöst. Die Unteransprüche kennzeichnen Ausgestaltungen mit dem die Branchen der Greifeinrichtung und die Schneideeinrichtung stabil und verdrehsicher geführt werden (Anspruch 2), durch die spezielle messerförmige Formgebung der Schneideelektrode wird die Gewebedurchtrennung zusätzlich erleichtert (Anspruch 3). Die beiden Schenkelbereiche der Koagulationslinien können gerade, gekrümmt oder wellenförmig (Anspruch 4) verlaufen. Auf jeden Fall liegen die beiden Branchen im zusammengedrückten Zustand parallel zueinander, dabei sind beide Branchen gerade gestreckt oder verlaufen gekrümmt (Anspruch 5). Zum sicheren Greifen des Gewebes ist häufig von Vorteil, wenn eine der beiden Branchen zur andern hin abgewinkelt ist und über das Ende der andern im zusammengeklappten Zustand hinwegragt. Ist eine der beiden Branchen derart hakenförmig abgewinkelt, daß im zusammengeklappten Zustand die hakenförmige Branche die andere überlappt, dann ist gerade durch diese Hakenform eine ausgezeichnete Greifeinrichtung für Gefäß und Gewebe gegeben, die bei bisher bekannten Zangen nur durch die Riffelung der Branchen, die gerne gegriffenes Gewebe verletzt, erreicht wird.

Der Vorteil liegt darin, daß neben dem herkömmlichen Greif- und Koagulationsvorgang auch die Gewebedurchtrennung über Hochfrequenzeinwirkung mit der dazu ausgebildeten Schneideelektrode erfolgt. Das ist insbesondere bei kleinen Durchtrennungslängen (< 5 mm) eine große Erleichterung, da ein Messerschnitt mit größerer Toleranz geführt werden muß, weil er nicht so sicher geführt werden kann und durch das Nach-Vorwärtsschieben des Messers nicht deutlich eingesehen werden kann. Der Chirurg ist somit kräftemäßig entlastet und kann sich voll auf die Führung des kombinierten Instrumentes richten.

Durch die linienförmige Ausgestaltung der beiden Zangenbranchen kann eine entsprechend beliebige, sinnvollerweise nach vorne geschlossene Koagulationslinie ins gegriffene Gewebe eingeprägt und dann der umfaßte Bereich vollends durchtrennt werden. Dabei können die beiden Branchen im zusammengeklappten Zustand in ihrem Schenkelbereich gestreckt, gekrümmt oder für beide Formen dazu noch wellenlinienförmig verlaufen. In der Form der Branchen ist eine sinnvolle Vielfalt möglich.

Die Erfindung wird im folgenden anhand der Zeichnung näher beschrieben. Es zeigt:
Figur 1 das gesamte Instrument,
Figur 2 die Ausgestaltung der Greif- und Schneideeinrichtung,
Figur 3 der geschlossene und geöffnete Greifer,
Figur 4 die Schneideelektrode in geschlossener und geöffneter Position,
Figur 5 die Zunge im Schaftende als Führungselement,
Figur 6 das distale Schaftende in der Draufsicht und
Figur 7 das abgewinkelte distale Ende in Draufsicht.

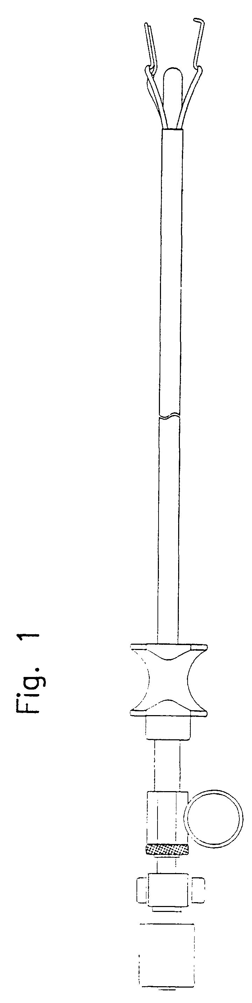

Das gesamte Instrument ist mit seiner Kontur in Figur 1 dargestellt. Links in der Figur ist der Bedienteil mit dem elektrischen Anschluß für den Hochfrequenzstrom. Der mittlere Bereich stellt das Teleskoprohr, den Schaft, dar, durch das hindurch die Übertragungselemente und eventuell sonstige für die Operation notwendige Einrichtungen geführt sind. Am rechten Bildteil ist die Greifeinrichtung, die Zange, mit Schneideeinrichtung und Führungszunge dargestellt.

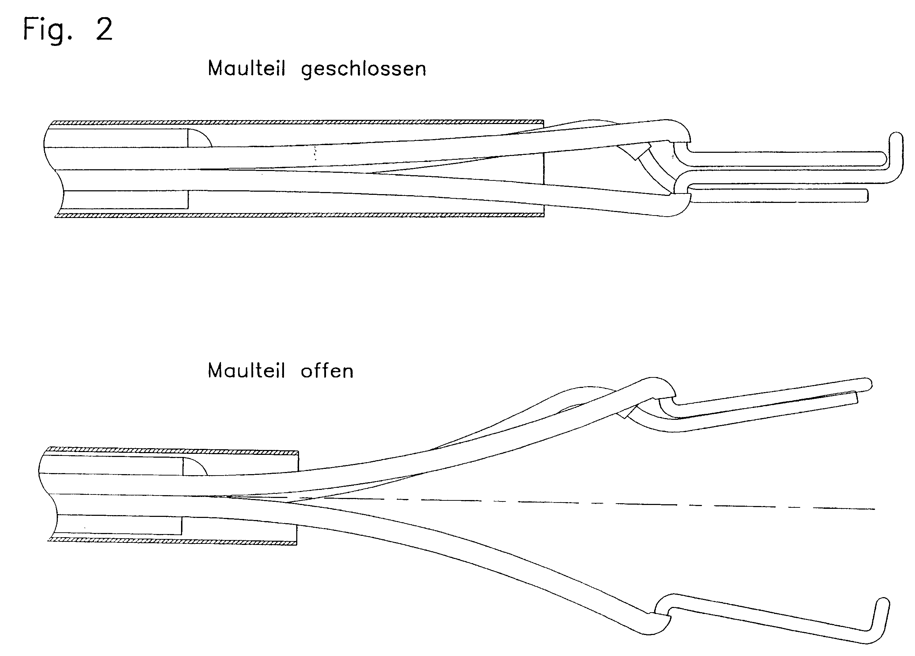

Figur 2 zeigt das Instrument in seinen beiden Endpositionen: oben im geschlossenen Zustand, unten im völlig geöffneten. Der Übersichtlichkeit halber ist die im distalen Schaftende fest verankerte Führungszunge weggelassen. Im geöffneten Zustand wird die Zange über das zu greifende Gewebe gehalten, dann wird das auf dem Schaftende axial verschiebbare Rohrteil (Hülse) vorgeschoben, um die beiden Branchen aufeiander zuzubewegen und das Gewebe zu greifen und schließlich entsprechend der Linienkontur der beiden Branchen zu koagulieren. Danach erst wird das Rohrteil noch weiter nach vorne geschoben, um die Schneideelektrode durch den von der Koagulationslinie umrandeten Gewebebereich zu führen und damit dort zu durchtrennen.

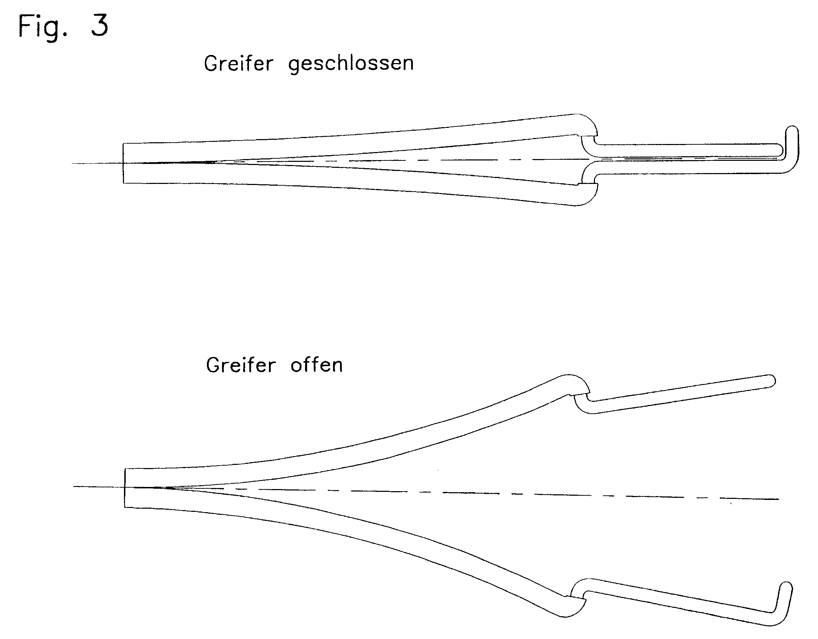

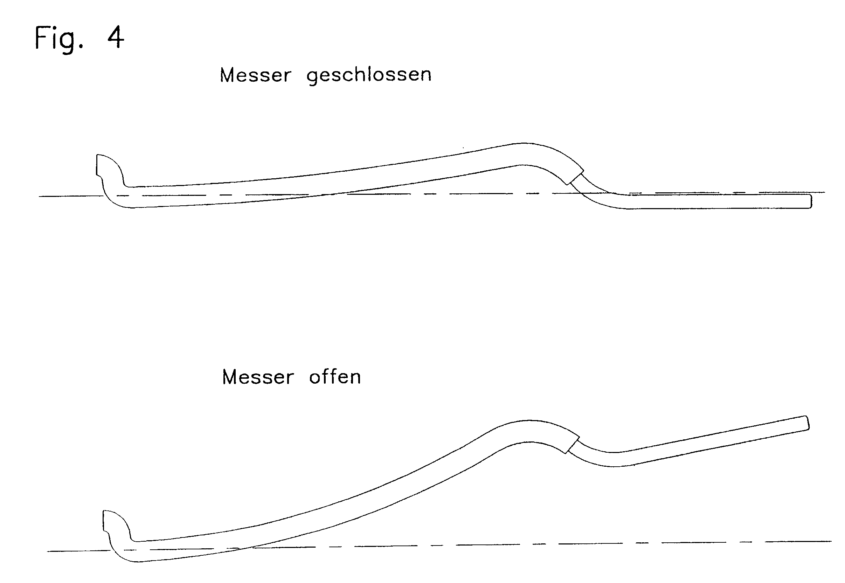

Die beiden Branchenformen und die bezüglich der Schaftachse spiegelbildliche, unidirektionale Krümmung der zugehörigen Führungsdrähte ist in Figur 3 skizziert, oben im geschlossenen und unten im geöffneten Zustand. Hier wird deutlich, das diese Branchenform das gegriffene Gewebe sicher greift. Figur 4 zeigt die Schneideeleektrode bezüglich der Schaftachse in den beiden Endstellungen.

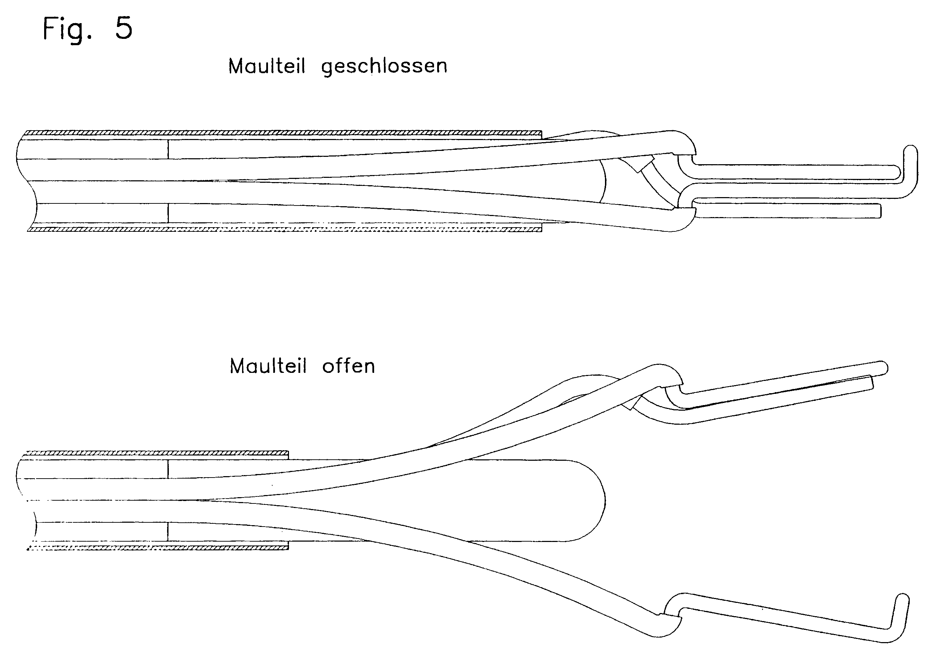

In der Figur 5 ist der Endbereich des distalen Schaftendes aufgeschnitten, um die Führungseigenschaft der starr eingebauten Führungszunge hervorzuheben. Hier werden beide Drähte für die Branchen diesseits der Zunge geführt, der Draht für die Schneideelektrode jenseits.

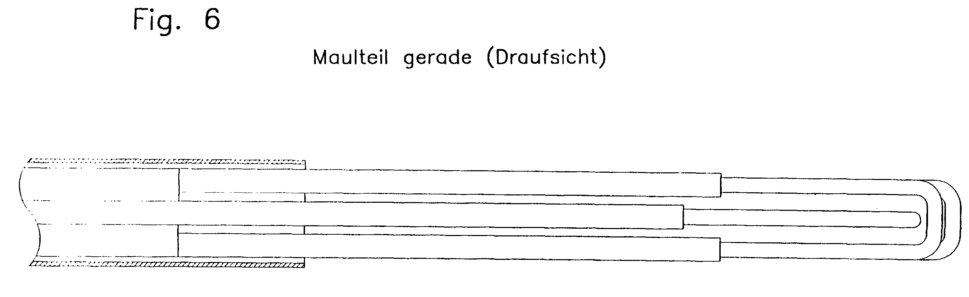

Die einfache linienförmige Gestaltung der beiden Branchen kommt in Figur 6 zum Ausdruck. Das ist nur eine von vielen möglichen, nach vorne geschlossenen Formen. Die Schneideelektrode liegt in der Mitte. Das ist zwingend, da ja nur zwischen der nach vorne geschlossenen Koagulationslinie der Durchtrennungsschnitt geführt werden darf.

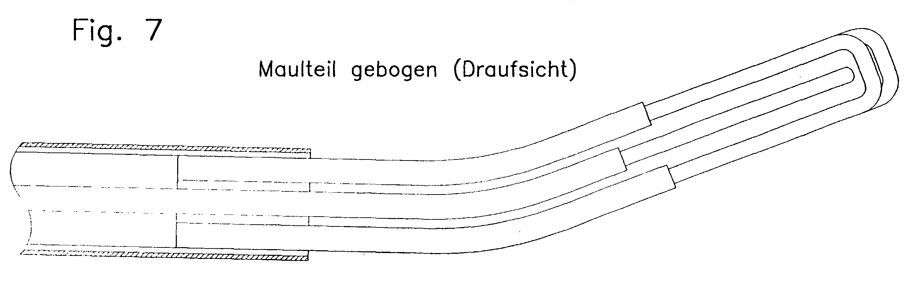

Figur 7 zeigt den abgeknickten Aktionsbereich des Instruments, bei dem der Arbeitsbereich neben der Schaftachse liegt und somit von dem Chirurgen gut eingesehen werden kann.

Die Materialien - metallischer als auch nichtmetallischer Art - aus denen das Instrument besteht, sind gewebeverträgliche und für den medizinischen Bereich zugelassene und taugliche, insbesondere auch in Hinblick auf Reinigung und Sterilisation. Des weiteren ist der elektrotechnische Aufbau gegenüber dem Patienten derart abgeschlossen, daß kein Stromfluß durch ihn hindurch eintritt.

## Patentansprüche

1. Bipolares Koagulations- und Schneidegerät für die endoskopische Chirurgie, bestehend aus:
einem Betätigungsteil, einem Schaftteil, einer Greifeinrichtung zum Greifen von Gewebe, die aus zwei aufeinander zu- und voneinander wegbewegbaren Branchen besteht, die über die axiale Bewegung, des Schaftes oder eines am Schaftende aufgesetzten Rohrteils in Form einer Hülse bewegt werden, mit denen bipolar koaguliert wird, und einer zwischen den beiden Branchen geführten Schneideeinrichtung, wobei jede Branche am gekrümmten Ende eines elastischen Drahts ansetzt und die Drähte spiegelbildlich zur Schaftachse, gekrümmt sind, und einem dritten, ebenfalls im Schaft verlaufenden und dort entsprechend wie einer der beiden Branchendrähte stärker gekrümmten Drahte,
**dadurch gekennzeichnet, daß** die Drähte
mit gleichbleibender Krümmungsrichtung gekrümmt sind und im Schaft verlaufen,
am Ende des dritten Drahtes eine axial ausgerichtete Drahtelektrode ansetzt, die bei zusammengedrückten Branchen durch weiteres Vorführen der Hülse durch die Greifeinrichtung gefahren wird und.dabei das gegriffene, vorher mit den Branchen schon koagulierte Gewebe mit Hilfe eines Hochfrequenzstromes durchtrennt.

2. Bipolares Koagulations- und Schneidegerät nach Anspruch 1, **dadurch gekennzeichnet, daß**
aus dem distalen Schaftende eine im Schaft festsitzende Zunge bis höchstens zu den Drahtenden herausragt und damit die drei Drähte führt und bei gegriffenem Gewebe gegen Verdrehen sichert.

3. Bipolares Koagulations- und Schneidegerät nach Anspruch 2, **dadurch gekennzeichnet, daß**
die Drahtelektrode in ihrer Durchtrennrichtung messerformig angeschliffen ist.

4. Bipolares Koagulations- und Schneidegerät nach Anspruch 3, **dadurch gekennzeichnet, daß**
die Schenkel der beiden Branchen wellenförmig verlaufen und die beiden Schenkel einer Branche spiegelbildlich zu denen der andern verlaufen.

5. Bipolares Koagulations- und Schneidegerät nach Anspruch 4, **dadurch gekennzeichnet, daß**
die jeweiligen Achsen der beiden Branchen im zusammengedrückten Zustand parallel verlaufen und gerade oder gekrümmt sind.

6. Bipolares Koagulations- und Schneidegerät nach Anspruch 5, **dadurch gekennzeichnet, daß**
eine der beiden Branchen an ihrem Endbereich bis zu höchstens 90° zur andern Branche hin abgewinkelt ist und diese im zusammengedrückten Zustand überlappt.

## Claims

1. Bipolar coagulation and cutting device for endoscopic surgery, comprising:
an actuating portion, a shaft portion, a gripping device for gripping tissue, said gripping device comprising two branches which can be displaced towards each other and can be displaced away from each other and are moved via the axial movement of the shaft or of a tube portion in the form of a sleeve at the end of the shaft, with which branches bipolar coagulation is effected, said bipolar coagulation and cutting device also comprising a cutting device which is guided between the two branches, each branch attaching to the curved end of a resilient wire and the wires being curved in a mirror-image manner relative to the shaft axis, and a third wire, which also extends in the shaft and is correspondingly more strongly curved in the shaft than one of the two branch wires, **characterised in that** the wires are curved with a constant direction of curvature and extend in the shaft, at the end of the third wire is attached an axially orientated wire electrode, which, when the branches are pressed together, is moved through the gripping means by advancing the sleeve further and at the same time, by means of a high frequency current, cuts through the gripped tissue, which has already been coagulated beforehand by means of the branches.

2. Bipolar coagulation and cutting device according to claim 1, **characterised in that** from the distal end of the shaft a tongue, which is fixedly secured in the shaft, projects a maximum of as far as the ends of the wires and consequently guides the three wires and secures them from twisting when tissue is gripped.

3. Bipolar coagulation and cutting device according to claim 2, **characterised in that** the wire electrode acts in the form of a knife in its cutting-through direction.

4. Bipolar coagulation and cutting device according to claim 3, **characterised in that** the portions of the two branches are wave-like in shape and the two portions of one branch extend in a mirror-image manner relative to those of the other.

5. Bipolar coagulation and cutting device according to claim 4, **characterised in that** the respective axes of the two branches in the pressed-together condition extend in parallel and are straight or curved.

6. Bipolar coagulation and cutting device according to claim 5, **characterised in that** one of the two branches is angled at its end region up to a maximum of 90° relative to the other branch and overlaps this latter in the pressed-together condition.

## Revendications

1. Appareil bipolaire de coagulation et de coupe destiné à la chirurgie endoscopique, composé
d'une partie de commande, d'une partie de queue, d'une installation de saisie destinée à saisir des tissus et composée de deux branches qu'on peut rapprocher et éloigner l'une de l'autre et qui se déplacent grâce au mouvement axial de la queue ou d'une partie tubulaire placée à l'extrémité de la queue sous forme d'un manchon, et grâce auxquelles on réalise la coagulation bipolaire, et d'une installation de coupe guidée entre les deux branches, chaque branche s'appliquant à l'extrémité courbée d'un fil métallique élastique et les fils métalliques étant recourbés en symétrie bilatérale vers l'axe de la queue, et d'un troisième fil métallique qui s'étend également dans la queue et qui y est plus fortement recourbé tout comme l'un des deux fils métalliques de branche,
**caractérisé en ce que**
les fils métalliques sont recourbés en conservant leur direction de courbure et s'étendent dans la queue,
à l'extrémité du troisième fil métallique s'applique un fil-électrode orienté axialement qui est déplacé à travers l'installation de saisie grâce à une autre avancée du manchon lorsque les branches sont rassemblées et le tissu, saisi et préalablement coagulé par les branches, est alors sectionné à l'aide d'un courant à haute fréquence.

2. Appareil bipolaire de coagulation et de coupe selon la revendication 1,
**caractérisé en ce qu'**
une languette bloquée dans la queue dépasse de l'extrémité distale de queue tout au plus jusqu'aux extrémités des fils métalliques et par conséquent guide les trois fils métalliques et les protège de la torsion lorsque le tissu est saisi.

3. Appareil bipolaire de coagulation et de coupe selon la revendication 2,
**caractérisé en ce que**
dans sa direction de sectionnement, le fil-électrode est affûté en forme de couteau.

4. Appareil bipolaire de coagulation et de coupe selon la revendication 3,
**caractérisé en ce que**
les côtés latéraux des deux branches ont une forme ondulée et les deux côtés latéraux d'une branche ont une symétrie bilatérale par rapport à ceux de l'autre branche.

5. Appareil bipolaire de coagulation et de coupe selon la revendication 4,
**caractérisé en ce que**
à l'état replié, les axes des deux branches sont parallèles et sont rectilignes ou courbés.

6. Appareil bipolaire de coagulation et de coupe selon la revendication 5,
**caractérisé en ce que**
l'une des deux branches à son extrémité terminale est coudée tout au plus de 90° par rapport à l'autre branche et la chevauche à l'état rassemblé.
